# EUROPEAN PATENT APPLICATION

(11) **EP 3 167 828 A1**
(43) Date of publication of application: **17.05.2017**
(21) Application number: 16198420.8
(22) Date of filing: 11.11.2016
(51) Int. Cl.: A61B 17/34, A61B 17/00

(54) **APPARATUS FOR USE IN CONJUNCTION WITH AN INTRAOSSEOUS DEVICE**

(30) Priority: 15.11.2015 IL 24259415
(71) Applicant: Waismed Ltd, 4809234 Rosh Ha'Ayin (IL)
(72) Inventor: Ben-Mocha, Moshe, 6918607 Tel-Aviv (IL); Swisa, Einat, 4291000 Avihayil (IL)
(74) Representative: Graf von Stosch, Andreas

(57) **Abstract**

Apparatus for use in conjunction with an intraosseous device comprises a skin engageable plate formed with an aperture adjoining a skin site to be penetrated by an intraosseous device and with a pointer which is unmistakenly indicative of a location of a prominent or protruding anatomical feature and which is sized such that a distance from a terminal end of the pointer to the site-adjoining aperture corresponds to patient-specific dimensions from the anatomical feature to the penetration site; and an annular guide channel surrounding the site-adjoining aperture and protruding proximally from the skin engageable plate, for stabilizing a stylet which is introducible through the aperture to penetrate an adjoining anatomical structure along a confirmed path of penetration coinciding with the penetration site into a selected bone while producing a substantially uniform bore at the penetration site as it is advancing along the path of penetration.

## Description

### Field of the Invention

The present invention relates to the field of intraosseous devices for accessing bone marrow. More particularly, the invention relates to apparatus used in conjunction with an intraosseous device which facilitates accurate advancement, including manual and controllable advancement, of a needle through anatomical structures in order to access the bone marrow.

### Background of the Invention

The administration of medication to an injured or critically ill patient is many times delayed due to the difficulty in establishing an intravenous line. During such situations, a lifesaving alternative by which vascular access is quickly achieved is through intraosseous (IO) infusion, whereby fluids and medications are injected into a marrow cavity of a long bone such as the femur, tibia and humerus drain into a central venous canal, and are then carried to the bloodstream.

The success of an IO infusion procedure is contingent upon the accurate penetration of the bone cortex in order to access the bone marrow. Inaccurate penetration of an anatomical structure leads to many complications, including extravasation of fluid whereby infused fluid enters an extravascular space or tissue around the penetration site. If left untreated, the fluid accumulation may lead to a compartment syndrome and the associated risk of the loss of a limb. Extravasation typically occurs as a result of needle misplacement in the bone or excessive movement of the needle during the penetration procedure leading to enlargement of the penetration site relative to the diameter of the needle.

Particularly, the bones of infants are very thin and are sometimes concealed by excessive overlying soft tissue. A health practitioner performing an IO penetration procedure therefore requires a high level of accuracy to locate the bone and to identify the proper penetration site for the IO needle. Due to the thinness of the bone, many attempts to access the bone marrow fail as a result of incorrectly inserting the needle of an IO device, such as inserting the needle into a muscle and not into the bone. At times, the anatomical site for the needle insertion is incorrectly identified.

Many manual IO devices or automatic IO devices, i.e. loaded or power driven, for accessing bone marrow are known from the prior art, by which a rotating needle penetrates the bone marrow cavity. All of these prior art devices suffer from the drawback of excessive needle movement during the penetration procedure.

US 2010/0298784, for example, discloses an apparatus for manually penetrating bone in order to access the bone marrow. The apparatus has a handle with a drive shaft, a connector having a first end operable to connect to the drive shaft and a second end operable to attach to a penetrator hub, which may include a penetrator operable to access the bone marrow. The handle is manually rotated in order to rotate the connector and penetrator and to insert the penetrator into the bone and bone marrow.

During rotation of the penetrator, the health practitioner at times notices that the penetrator is not advancing through the skin at a correct angle. The inaccurate penetration is corrected by first rotating the handle back and forth in alternating rotational directions, or by displacing the handle back and forth in alternating lateral directions, and then slightly changing the penetration angle. The bore at the penetration site is enlarged as a result of the application of a bodily force onto the tip of the penetrator and is therefore not uniform due to the change in direction of the penetrator, causing bacterial ingress and infection thereat and possibly leading to the compartment syndrome.

Another difficulty faced by the health practitioner during an IO penetration procedure is the accurate and speedy identification of the penetration site.

US 7,347,840 discloses a template patch for locating the target site for intraosseous fluid infusion and aspiration. The template patch uses a key anatomical feature of a bone as a reference point to a target zone for infusion that is located a predetermined distance away from the feature. The apparatus also comprises a housing assembly with inner and outer sleeves, a spring assembly, a bone probe assembly, a release mechanism and a coupler for coupling a force applied by a user to drive the bone portal into the bone marrow via the target zone to a predetermined depth. Following withdrawal of the housing, the bone portal remains embedded in the bone marrow and an infusion tube extends out of the skin.

Although the penetration site can be identified, the needle often penetrates the anatomical structure at an incorrect angle and the penetration site is subsequently enlarged in an attempt to correct the path of penetration, leading to the complications described above.

It is an object of the present invention to provide apparatus for use in conjunction with an IO device that maintains the same angle of penetration from the skin to the bone.

It is an object of the present invention to provide apparatus for use in conjunction with an IO device that assures a uniform bore to be produced at the penetration site, to prevent fluid extravasation and to minimize the ingress of infection.

It is an object of the present invention to provide a manual IO device that facilitates control in the depth of penetration and in the formation of an accurate and uniform bore diameter within the bone.

It is yet an additional object of the present invention to provide an IO device by which the penetration site can be accurately and speedily identified with reference to an easy to palpate anatomical site.

Other objects and advantages of the invention will become apparent as the description proceeds.

### Summary of the Invention

The present invention provides a manually advanceable intraosseous device, comprising a skin engageable plate formed with an aperture adjoining a skin site to be penetrated and with a pointer which is unmistakenly indicative of a location of a prominent or protruding anatomical feature and which is sized such that a distance from a terminal end of said pointer to said site-adjoining aperture corresponds to age or size specific dimensions from said anatomical feature to said penetration site; a cannula unit to which a distally extending cannula is secured; a handle releasably engageable with said cannula unit for manually manipulating said cannula unit; a coupler extending proximally and fixedly from said plate; one or more engagement elements provided with said coupler for cooperating with said cannula unit in such a way that displacement of said handle in response to manual manipulation thereof is converted to differential and controllable axial advancement of said cannula unit, so that said cannula in conjunction with a stylet receivable therewithin is introducible through said aperture to penetrate an adjoining anatomical structure along a confirmed path of penetration coinciding with said penetration site into a selected bone while producing a substantially uniform bore at said penetration site; and an annular guide channel surrounding said site-adjoining aperture and protruding proximally from said skin engageable plate, for stabilizing said stylet as it is advancing along said path of penetration.

The engagement elements cooperating with the cannula unit are configured in accordance with the structure of the coupler and with the intended manner of manual manipulation, for example threading interengageable with threading formed on the cannula unit when the coupler is an annular wall and the manual manipulation is rotational movement or one or more linearly shaped engagement elements when the manual manipulation includes an axial movement.

In one aspect, the coupler is an annular wall and the one or more engagement elements provided with the coupler are configured by threading formed in an inner surface of said annular wall which are interengageable with threading formed on the cannula unit.

In one aspect, the coupler is integrally formed with the skin engageable plate.

In one aspect, the coupler is releasably attachable to the skin engageable plate.

In one aspect, the skin engageable plate is circular and the pointer tangentially extends from a peripheral region of the plate, for aligning the aperture with the penetration site.

In one aspect, the cannula unit comprises a distal hub interengageable with the coupler and a proximal throat of a considerably smaller diameter than that of said hub, wherein said hub has a solid interior which is formed with a central bore capable of being collinear with the guide channel for increased stylet stabilization.

In one aspect, the handle is releasably engageable with the throat of the cannula unit and has a cavity for receiving a proximal end of a trocar secured to the stylet.

In one aspect, a proximal surface of the handle is formed with one or more notched regions through which the proximal end of the trocar is laterally displaceable. The handle is laterally releasable from the cannula unit while the proximal end of the trocar is located within one of the notched regions.

The invention is also directed to an apparatus for use in conjunction with an intraosseous device, comprising a skin engageable plate formed with an aperture adjoining a skin site to be penetrated by an intraosseous device and with a pointer which is unmistakenly indicative of a location of a prominent or protruding anatomical feature and which is sized such that a distance from a terminal end of said pointer to said site-adjoining aperture corresponds to patient-specific dimensions from said anatomical feature to said penetration site; and an annular guide channel surrounding said site-adjoining aperture and protruding proximally from said skin engageable plate, for stabilizing a stylet which is introducible through said aperture to penetrate an adjoining anatomical structure along a confirmed path of penetration coinciding with said penetration site into a selected bone while producing a substantially uniform bore at said penetration site as it is advancing along said path of penetration.

The intraosseous device may be manually manipulated, spring loaded or power driven.

### Brief Description of the Drawings

In the drawings:
- Fig. 1 is a perspective view from the side of an assembled intraosseous device, according to one embodiment of the invention;
- Fig. 2 is a perspective view from the top of components of the device of Fig. 1 when in a disassembled state;
- Fig. 3 is a perspective view from the top of a guide component used in conjunction with the device of Fig. 1;
- Fig. 4 illustrates the location by palpation of a suitable anatomical feature to be used a reference point for the device of Fig. 1;
- Fig. 5 illustrates the positioning of a pointer of the guide component of Fig. 3 at selected anatomical feature;
- Fig. 6 is a perspective view from the side of a cannula unit used in conjunction with the device of Fig. 1;
- Fig. 7 is a longitudinal cross sectional view of the cannula unit of Fig. 6;
- Fig. 8 is a top view of a handle used in conjunction with the device of Fig. 1;
- Fig. 9 is a cross sectional view of the handle of Fig. 8, cut along plane A-A;
- Fig. 10 is a cross sectional view of the handle of Fig. 8, cut along plane B-B;
- Fig. 11 is a longitudinal cross sectional view of the assembled intraosseous device of Fig. 1;
- Fig. 12 illustrates the feeding of a cannula into a guide channel following the positioning operation shown in Fig. 5;
- Fig. 13 illustrates the separation of the handle of Fig. 8 from the cannula unit of Fig. 6 by lateral displacement;
- Fig. 14 illustrates hand initiated removal of a trocar from the cannula unit of Fig. 6;
- Fig. 15 illustrates removal of a trocar from the cannula unit of Fig. 6 by means of an engagement element formed in a distal region of a handle, when inverted;
- Fig. 16 is an exploded perspective view of an intraosseous device, according to another embodiment of the invention; and
- Fig. 17 is a perspective view from the top of a guide component, according to another embodiment of the invention.

### Detailed Description of Preferred Embodiments

The present invention is novel apparatus for use in conjunction with an intraosseous (IO) device for controllably accessing bone marrow, by which a health practitioner, particularly a doctor due to the skill and expertise needed during a bone penetration operation, is able to accurately determine a correct penetration site, and in one aspect of the invention the depth of penetration within the bone. The IO device of the present invention is suitable for, but not limited to, penetrating the bone marrow cavity in the proximal tibia, based on the tibial tuberosity as an anatomical mark.

As opposed to prior art IO devices which are manually or automatically rotated during penetration into the bone and the bone marrow cavity along a user selected path and which at times require the path of penetration to be adjusted if the penetrator is found not be advancing in an optimal fashion, producing a non-uniform and unsightly bore in the skin or bone that leads to bacterial ingress, the apparatus of the present invention comprises a guide that directs the intraosseous needle, hereinafter referred to as a "stylet", along an anatomically confirmed path of penetration into a bone to produce a substantially uniform bore.

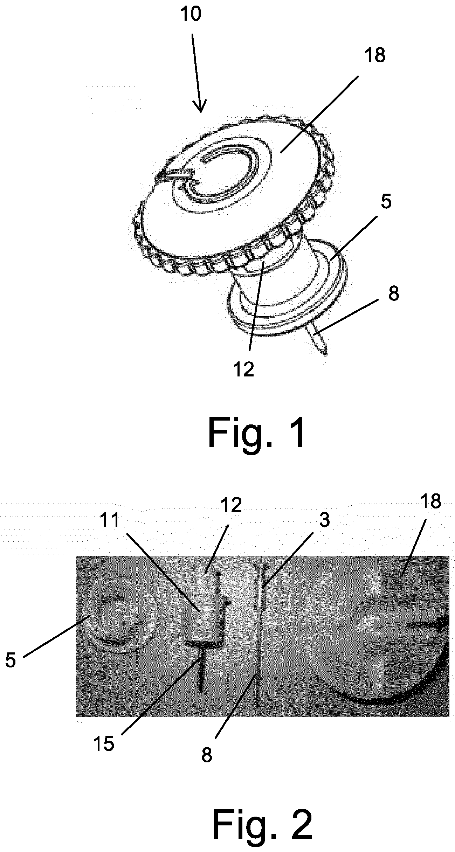

Fig. 1 illustrates an assembled IO device, generally indicated by numeral 10, according to one embodiment of the present invention. IO device 10 comprises stabilizing guide 5, which is positionable in abutting relation with the skin of a patient in need of an IO infusion or of a bone marrow biopsy, at a predetermined distance from a prominent or protruding anatomical feature that is conclusively indicative of the preferred path of penetration into the adjacent bone. A stylet 8 is received within cannula unit (CU) 12, and CU 12 is releasably and movably engaged with guide 5. By suitable manipulation of handle 18, which is circular or configured in any other ergonomic fashion and is releasably engaged with CU 12, stylet 8 is able to protrude distally beyond guide 5 towards the bone desired to be penetrated.

The aforementioned components of IO device 10 are illustrated in Fig. 2 when in a disassembled state. CU 12 is shown to have a metallic cannula 15 that distally extends from a hub, which may be made of a plastic material. Stylet 8 of trocar 3 is insertable within the lumen of cannula 15.

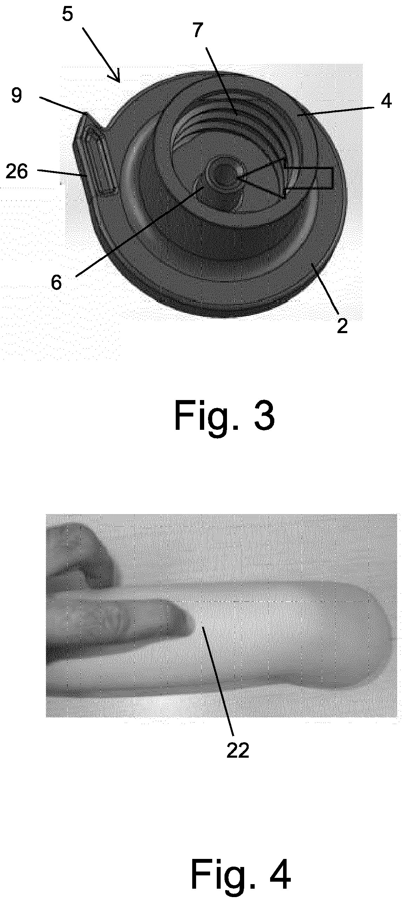

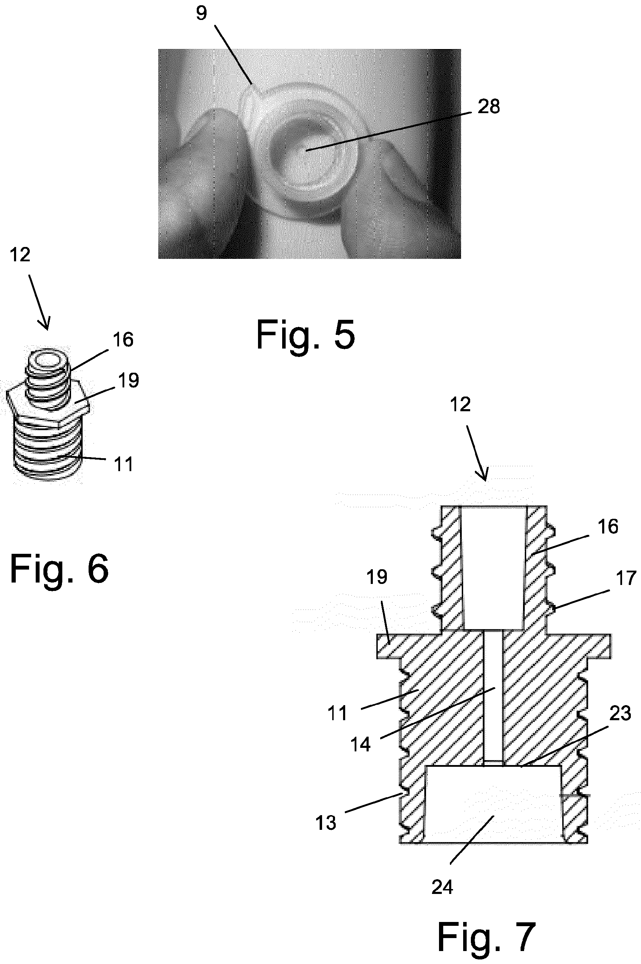

Guide 5 shown in Fig. 3 is configured with a circular skin engaging plate 2 which is formed with an aperture 28 (Fig. 5) adapted to coincide with a selected penetration site. An annular guide channel 6 through which the stylet is inserted and which surrounds the site-adjoining aperture may protrude proximally from plate 2, and serves to stabilize the stylet as the latter is being advanced towards the bone marrow due to the relatively long channel length and the small clearance between the stylet and channel. A coupler 4 which also protrudes proximally from, and is integrally formed with, plate 2 concentrically surrounds both the site-adjoining aperture and guide channel 6. Coupler 4 is formed with internal helical threading 7 by which the cannula unit is releasably and movably engaged with guide 5.

Coupler 4 and guide channel 6 are shown to proximally extend from plate 2 at an angle of 90 degrees. It will be appreciated, however, that one or both of coupler 4 and guide channel 6 may proximally extend from plate 2 at any other desired angle, such as 30 or 60 degrees, to take into account anatomical considerations of the anatomical structure proximate to the penetration site.

An extension 26 which terminates with a pointer 9 tangentially extending from the periphery of plate 2 is used to properly align guide channel 6 with the penetration site. The length of extension 26 and the distance of pointer 9 from guide channel 6 are selected to correspond with patient-specific dimensions from an anatomical feature to the penetration site, from which the stylet is advanceable subcutaneously along a preferred path of penetration and then intraosseously so as to penetrate a given bone.

For example, as shown in Fig. 4, a suitable anatomical feature to be used as a reference point for positioning the guide channel is the tibial tuberosity 22 located on the proximal tibia. This anatomical feature is able to be located by palpation. Pointer 9 is then positioned at the located tibial tuberosity, as shown in Fig. 5 while the extension is parallel to the orientation of the proximal tibia, so that site-adjoining aperture 28, and therefore the stylet, will be suitably aligned with the preferred path of penetration. A penetration site aligned with aperture 28 is assured of being sufficiently spaced from the tibial tuberosity to avoid injury to the epiphyseal growth plate and to direct the stylet to a location that is known to have a best success rate for penetrating the bone marrow cavity, based on the patient's age. Validation of the dimensions of pointer 9 and extension 26 is based on the analysis of a previously scanned CT image.

It will be appreciated that any other pointer configuration which is unmistakenly indicative of a location of a prominent or protruding anatomical feature is also in the scope of the invention.

CU 12 is illustrated in Figs. 6 and 7. CU 12 has a distal hub 11 formed with threading 13 that is recessed from its outer surface. Threading 13 is engageable with threading 7 formed in the inner surface of coupler 4 (Fig. 3), to facilitate axial displacement of CU 12. CU 12 also has a proximal annular throat 16 having a significantly smaller diameter than hub 11. Throat 16 is formed with outer threading 17 for engagement with an infusion set. Abutment 19, which may be hexagonal, separates hub 11 from throat 16 and radially protrudes from hub 11.

The solid interior of hub 11 has a central bore 14 into which the stylet is insertable. Bore 14 is adapted to be collinear with guide channel 6 of guide 5 (Fig. 3), and serves as additional means for stabilizing the stylet. Hub 11 also has a distal cavity 24 in communication with, and which may be of a significantly larger diameter than, bore 14. Guide channel 6 is adapted to be received in cavity 24 and in abutting relation with proximal cavity edge 23 when CU 12 is movably engaged to a fullest extent with respect to guide 5.

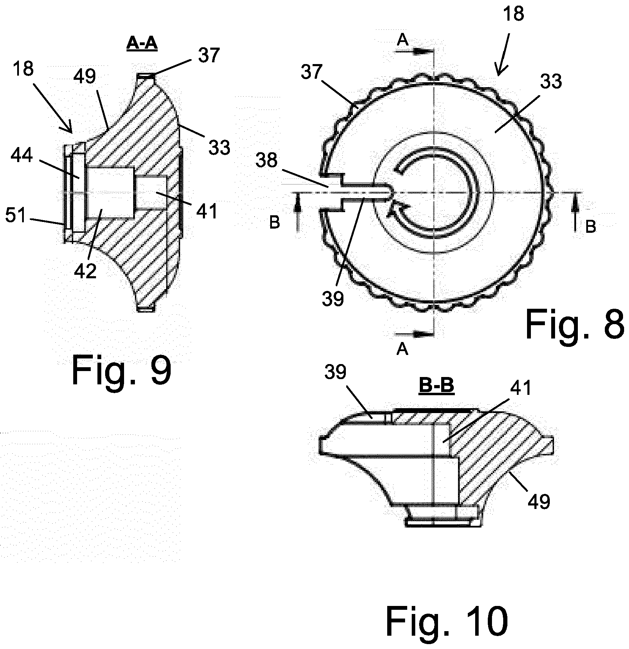

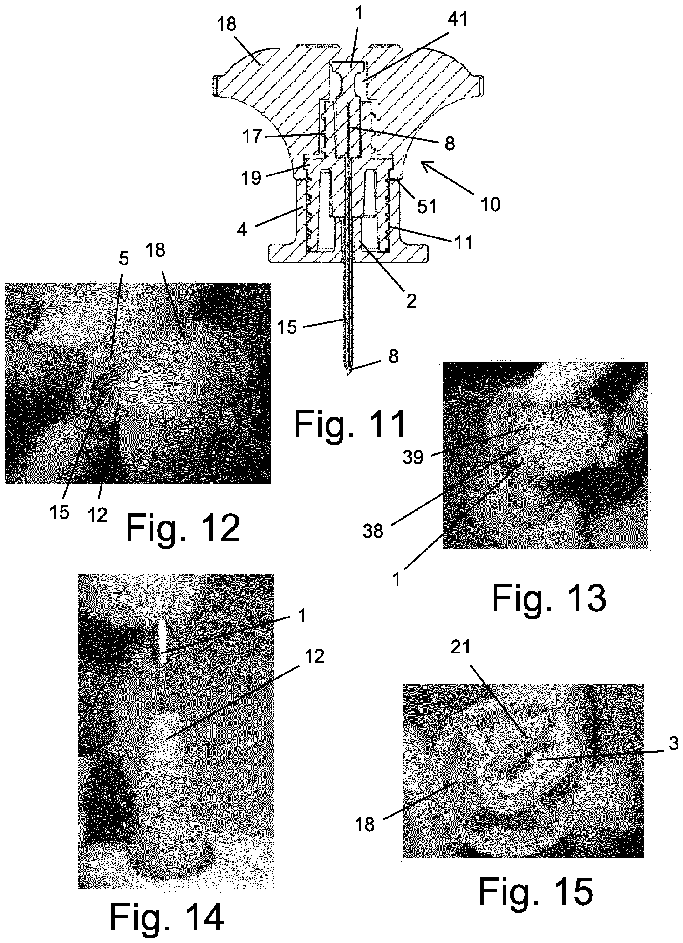

Handle 18 is illustrated in Figs. 8-10, and is shown to be configured with a proximal circular surface 33 that is slightly convex and with a plurality of circumferentially spaced grip portions 37 disposed outwardly from proximal surface 33, to assist in performing a penetrating operation. Proximal surface 33 is formed with two notched portions 38 and 39 arranged such that portion 38 is removed from the outer periphery of surface 33 and portion 39 is narrower and more inwardly disposed than portion 38. Notched portion 39, which is in communication with cavity 41 distally spaced from proximal surface 33, is sized to receive the proximal end 1 of the trocar (Fig. 11), which is secured to stylet 8, so that it will be allowed to be positioned within cavity 41. A slightly larger cavity 42 formed distally to cavity 41 and concentric to the central axis of handle 18 is adapted to receive throat 16 of CU 12. A wider cavity 44 and of a smaller height is formed distally to cavity 42, and is adapted to receive abutment 19 of CU 12. Handle 18 also has an outer distal surface 49 which is concave to allow the width of handle 18 to be gradually reduced from a maximum value at the grip portions 37 to a minimum value at distal edge 51, so that distal edge 51 can be brought in abutting relation with coupler 4 of guide 5 when the handle is rotated to a maximum extent.

It will be appreciated that the notched handle may be configured without the outer distal surface.

Fig. 11 illustrates a cross sectional view of the assembled IO device 10. Although the tip of stylet 8 is shown to protrude only slightly from the distal end of cannula 15, many other configurations are envisioned, including one wherein the stylet significantly protrudes from the cannula, yet is advantageously prevented from being misshaped by the stabilizing effect provided by guide channel 2 and the bore formed in hub 11.

In operation, after the proximal end 1 of the trocar is positioned within cavity 41 of handle 18 and cannula 15 is inserted within, and adhesively affixed to the wall of, bore 14 of CU 12 (Fig. 7) such that it distally extends from cavity 24, throat 16 of CU 12 is positioned in proximity to handle 18. Stylet 8 is fed through the adhesively affixed cannula 15 and then CU 12 is laterally displaced so that the proximal end 1 of the trocar passes through the notched portions of handle and abutment 19 is received in cavity 44.

As shown in Fig. 12, cannula 15 extending from the coupled CU 12 and handle 18 is fed into guide channel 6 after guide 5 has been skin engaged at a predetermined distance from a known anatomical feature by pressing the skin engageable plate to the skin and positioning the pointer at the anatomical feature, as described above. Guide channel 6 accurately directs cannula 15 generally but not necessarily perpendicularly into the skin, along an anatomically confirmed path of penetration into a desired bone. The length of cannula 15 and of the stylet protruding therefrom is sufficient to penetrate the desired bone, in order to access the bone marrow cavity. Handle 18 is then rotated while hub 11 of CU 12 is threadedly engaged with coupler 4 of guide 5. During the first subcutaneous penetration of 4-5 mm, handle 18 may be rotated back and forth in order to prevent the twisting of skin around the stylet tip. The handle may be rotated in the same rotational direction while the stylet tip is penetrating the soft tissues. By virtue of this threaded engagement, rotational movement of the hand manipulating handle 18 is able to be converted into precise and controllable axial movement of CU 12. The health practitioner, particularly an experienced doctor, is able to receive tactile feedback when the stylet enters the bone marrow cavity, as an indication when to terminate the penetration operation, for example to prevent penetration of the stylet into the opposite cortex.

Following termination of the penetration operation, handle 18 may be advantageously separated from CU 12 by being translated sideways in such a direction that the proximal end 1 of the trocar slides through notched portions 38 and 39, as shown in Fig. 13.

Trocar 3 provided with the stylet is then removed from the anatomical structure that it has penetrated and separated from CU 12 by holding its protruding proximal end 1 by hand and pulling as shown in Fig. 14, or by manipulating handle 18 when inverted to engage trocar 3 with a distal engagement element 21, e.g. U-shaped, formed in a distal location of handle 18, as shown in Fig. 15. The cannula remains in penetration to the anatomical structure, and is consequently used to infuse liquids to the bone marrow.

As may be appreciated, the stylet is advantageously removed prior to the infusion operation to provide a greater interior volume of the cannula into which the fluids are introducible by means of an IO-suitable infusion set, without concern that the stylet has to be reinserted at a different puncture site by virtue of the accuracy that IO device 10 is afforded in identifying the preferred path of penetration.

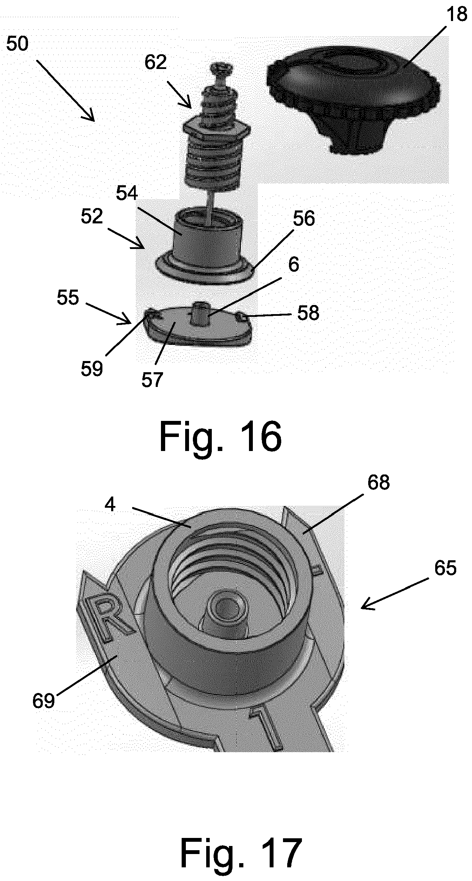

Fig. 16 illustrates another embodiment of the invention wherein IO device 50 is configured similarly to IO device 10 of Fig. 1, but with a releasably attachable coupler. Coupling unit 52 has an annular coupler 54 formed with helical threading recessed from its inner face for engagement with cannula unit 62, and a rim 56 surrounding, and integral with, coupler 54 at its distal end. Guide unit 55 has a circular skin engaging plate 57 which is provided with a central proximally extending guide channel 6 that surrounds the site-adjoining aperture, and two catches 58 and 59, e.g. diametrically opposed, which are slightly proximally separated from plate 57. Catch 59 may be configured as a pointer as well. When rim 56 of coupling unit 52 is fitted under, and frictionally engaged with, catches 58 and 59, coupler 54 is concentric with guide channel 6. Coupling unit 52 may be secured to guide unit 55 either before or after being initially threadedly engaged with cannula unit 62.

Guide 65 shown in Fig. 17 is configured similarly to guide 5 of Fig. 3, with the exception of two pointers 68 and 69 located at different sides of coupler 4. A first pointer is for use with the right leg and a second pointer is for use with the left leg.

Although the aforementioned description relates the guidance of a stylet received in a manually manipulated cannula unit through guide channel 6 (Fig. 3), the stylet received in a spring loaded or power driven cannula unit may similarly be stabilized by the guide channel during a penetration procedure.

While some embodiments of the invention have been described by way of illustration, it will be apparent that the invention can be carried out with many modifications, variations and adaptations, and with the use of numerous equivalents or alternative solutions that are within the scope of persons skilled in the art, without exceeding the scope of the claims.

## Claims

1. A manually advanceable intraosseous device, comprising:
a) a skin engageable plate formed with an aperture adjoining a skin site to be penetrated and with a pointer which is unmistakenly indicative of a location of a prominent or protruding anatomical feature and which is sized such that a distance from a terminal end of said pointer to said site-adjoining aperture corresponds to patient-specific dimensions from said anatomical feature to said penetration site;
b) a cannula unit to which a distally extending cannula is secured;
c) a handle releasably engageable with said cannula unit for manually manipulating said cannula unit;
d) a coupler extending proximally and fixedly from said plate;
e) one or more engagement elements provided with said coupler for cooperating with said cannula unit in such a way that displacement of said handle in response to manual manipulation thereof is converted to differential and controllable axial advancement of said cannula unit, so that said cannula in conjunction with a stylet receivable therewithin is introducible through said aperture to penetrate an adjoining anatomical structure along a confirmed path of penetration coinciding with said penetration site into a selected bone while producing a substantially uniform bore at said penetration site; and
f) an annular guide channel surrounding said site-adjoining aperture and protruding proximally from said skin engageable plate, for stabilizing said stylet as it is advancing along said path of penetration.

2. The device according to claim 1, wherein the coupler is an annular wall and the one or more engagement elements provided with the coupler are configured by threading formed in an inner surface of said annular wall which are interengageable with threading formed on the cannula unit.

3. The device according to claim 2, wherein the coupler is integrally formed with the skin engageable plate.

4. The device according to claim 2, wherein the coupler is releasably attachable to the skin engageable plate.

5. The device according to claim 1, wherein the skin engageable plate is circular and the pointer tangentially extends from a peripheral region of the plate, for aligning the aperture with the penetration site.

6. The device according to claim 1, wherein the cannula unit comprises a distal hub interengageable with the coupler and a proximal throat of a considerably smaller diameter than that of said hub, wherein said hub has a solid interior which is formed with a central bore capable of being collinear with the guide channel for increased stylet stabilization.

7. The device according to claim 6, wherein the handle is releasably engageable with the throat of the cannula unit and has a cavity for receiving a proximal end of a trocar secured to the stylet.

8. The device according to claim 7, wherein a proximal surface of the handle is formed with one or more notched regions through which the proximal end of the trocar is laterally displaceable.

9. The device according to claim 8, wherein the handle is laterally releasable from the cannula unit while the proximal end of the trocar is located within one of the notched regions.

10. Apparatus for use in conjunction with an intraosseous device, comprising:
a) a skin engageable plate formed with an aperture adjoining a skin site to be penetrated by an intraosseous device and with a pointer which is unmistakenly indicative of a location of a prominent or protruding anatomical feature and which is sized such that a distance from a terminal end of said pointer to said site-adjoining aperture corresponds to patient-specific dimensions from said anatomical feature to said penetration site; and
b) an annular guide channel surrounding said site-adjoining aperture and protruding proximally from said skin engageable plate, for stabilizing a stylet which is introducible through said aperture to penetrate an adjoining anatomical structure along a confirmed path of penetration coinciding with said penetration site into a selected bone while producing a substantially uniform bore at said penetration site as it is advancing along said path of penetration.
